# EUROPEAN PATENT SPECIFICATION

(11) **EP 1 675 944 B1**
(45) Date of publication and mention of the grant of the patent: **24.11.2010**
(21) Application number: 04794329.5
(22) Date of filing: 07.10.2004
(51) Int. Cl.: C12N 5/079

(54) **HUMAN CORNEAL ENDOTHELIAL CELLS AND METHODS OF OBTAINING AND CULTURING CELLS FOR CORNEAL CELL TRANSPLANTATION**
MENSCHLICHE HORNHAUTENDOTHELZELLEN UND VERFAHREN ZUR GEWINNUNG UND KULTIVIERUNG VON ZELLEN ZUR HORNHAUTZELLTRANSPLANTATION
CELLULES ENDOTHELIALES CORNEENNES HUMAINES ET PROCEDES D'OBTENTION DE CULTURE DES CELLULES POUR LEUR TRANSPLANTATION CORNEENNE

(30) Priority: 10.10.2003 US 510344 P
(43) Date of publication of application: 05.07.2006
(73) Proprietor: Lui, Ge Ming, Honolulu, HI 96813 (US); Cellular Bioengineering, Inc., Honolulu, HI 96826 (US)
(72) Inventor: LUI, Ge Ming, Honolulu, HI 96813 (US)
(74) Representative: Høiberg A/S
(86) International application number: PCT/US2004/032933
(87) International publication number: WO 2005/038015

(56) References cited:
- ENGELMANN K ET AL: "[Endothelial cell transplantation and growth behavior of the human corneal endothelium]" DER OPHTHALMOLOGE : ZEITSCHRIFT DER DEUTSCHEN OPHTHALMOLOGISCHEN GESELLSCHAFT. SEP 1999, vol. 96, no. 9, September 1999 (1999-09), pages 555-562, XP002312235 ISSN: 0941-293X
- MIYATA K ET AL: "Effect of donor age on morphologic variation of cultured human corneal endothelial cells." CORNEA. JAN 2001, vol. 20, no. 1, January 2001 (2001-01), pages 59-63, XP002312236 ISSN: 0277-3740
- DATABASE BIOSIS [Online] BIOSCIENCES INFORMATION SERVICE, PHILADELPHIA, PA, US; December 2002 (2002-12), AMANO SHIRO: "Transplantation of corneal endothelial cells." XP002312237 Database accession no. PREV200300187415 & NIPPON GANKA GAKKAI ZASSHI, vol. 106, no. 12, December 2002 (2002-12), pages 805-836, ISSN: 0029-0203

## Description

### BACKGROUND OF THE INVENTION

### 1. Field of Invention

This patent describes improved methods of dissecting, seeding and subsequent propagation of pure culture of human corneal endothelial cells on extracellular matrix.

### 2. Description of Prior Art

For various reasons, the corneal portions of eyes may need to be surgically repaired or replaced. For example, the cornea may become scratched or scarred or otherwise physically damaged, greatly hindering sight. The cornea is also subject to the effects of various degenerative diseases, mandating replacement if the patient is to have normal or even near normal vision.

The cornea of the human eye is a specialized structure made up of substantially parallel relatively compacted layers of tissue. The outermost or most superficial layer of the cornea is the epithelial layer. This is a protective layer of tissue which regenerates if injured. Moving inwardly in the eye is the base surface of the epithelial layer known as Bowman's membrane. Immediately adjacent the Bowman's membrane is the stroma of the cornea, which is an extra-cellular collagen architectural matrix with scattered keratocytic cells. The stroma layer is bounded at its deepest level by a cuticular, a cellular membrane, referred to as Descemet's membrane, which is followed by a monolayer of single cell thickness of specialized endothelial cells which forms the posterior surface of the cornea. The endothelial layer does not regenerate and when it is diseased, scratched or otherwise injured, it must be replaced.

In some animal species including human, the corneal endothelium does not normally replicate in vivo to replace cells lost due to injury or aging (Murphy C, et al., Invest. Ophthalmology Vis. Sci. 1984; 25:312-322; Laing R A, et al., Exp. Eye Res. 1976; 22:587-594). However, human corneal cells can be cultured in vitro with a growth factor-enriched, fetal calf serum-containing medium under normal tissue culture conditions (Baum JL, et al., Arch. Ophthalmol. 97:1136-1140, 1979; Engelmann K, et al., Invest. Ophthalmol. Vis. Sci. 29:1656-1662, 1998; Engelmann K, and Friedl P; In Vitro Cell Develop. Biol. 25:1065-1072, 1989). If the cultured cells can be utilized to replace the loss of corneal endothelial cells it will greatly enhance the donor pool of human corneas. This is important as one may be able to augment the donor corneas currently rejected for transplantation procedures due to inadequate endothelial cell counts (Gospodarowicz D, et al., Proc. Natl. Acad. Sci. (USA) 76:464-468, 1979; Gospodarowicz D, et al., Arch. Ophthalmol. 97:2163-2169, 1979). This pool of corneas, rejected due to low endothelial cell density, makes up to 30% of the total donated corneas annually (National Eye Institute: Summary report on the cornea task force. Invest Ophthalmol Vis Sci 12:391-397, 1973). Furthermore, a method to culture human corneal endothelial cells from a low initial density, and the ability to reseed the cells grown in vitro onto denuded corneal buttons, will enable the use of the recipient's own undamaged stroma for allo-cell and auto-stroma type of transplantation (Insler MS, and Lopez JG, Cornea 10:136-148, 1991).

Tissue culture techniques are being successfully used in developing tissue and organ equivalents. The basis for these techniques involve collagen matrix structures, which are capable of being remodeled into functional tissue and organs by employing the right combination of living cells, nutrients, and culturing conditions. Tissue equivalents have been described extensively in many patents, including U.S. Pat. Nos. 4,485,096; 4,485,097; 4,539,716; 4,546,500; 4,604,346; 4,837,379; and 5,827,641, all of which are incorporated herein by reference. One successful application of the tissue equivalent is the living skin equivalent, which has morphology similar to actual human skin. The living skin equivalent is composed of two layers: the upper portion is made of differentiated and stratified human epidermal keratinocytes that cover a thicker, lower layer of human dermal fibroblasts in a collagen matrix. Bell, et al., "Recipes for Reconstituting Skin," J. of Biochemical Engineering, 113:113-119 (1991).

Studies have been done on culturing corneal epithelial and endothelial cells. Xie, et al., "A simplified technique for the short-term tissue culture of rabbit corneal cells," In Vitro Cellular & Developmental Biology, 25:20-22 (1989) and Simmons, et al., "Corneal Epithelial Wound Cloture in Tissue Culture: An in vitro Model of Ocular Irritancy," Toxicology and Applied Pharmacology, 88:13-23 (1987).

ENGELMANN K ET AL: "[Endothelial cell transplantation and growth behaviour of the human corneal endothelium]" DER OPHTHALMOLOGE: ZEITSCHRIFT DER DEUTSCHEN OPHTHALMOLOGISCHEN GESELLSCHAFT. SEP 1999, vol. 96, no. 9, September 1999 (1999-09), pages 555-562, XP002312235 ISSN: 0941-293X, discloses the culturing of human corneal endothelial cells and a method of *in vivo* transplantation of these cells onto the corneal membrane.

MIYATA ET AL: "Effect of donor age on morphologic variation of cultured human corneal endothelial cells." CORNEA. JAN 2001, vol. 20, no. 1, January 2001 (2001-01), pages 59-63, XP002312236 ISSN: 0277-3740, discloses culturing of human corneal endothelial cells from donors of varying age and the effects thereof.

DATABASE BIOSIS [Online] BIOSCIENCES INFORMATION SERVICE, PHILADELPHIA, PA, US; December 2002 (2002-12), AMANO SHIRO: "Transplantation of corneal endothelial cells." XP002312237 Database accession no. PREV200300187415, discloses culturing of human corneal endothelial cells on extracellular matrix from calf corneal endothelial cells by using basic FGF and foetal bovine serum.

Until the advent of the present invention, prior art methods of culturing human corneal endothelial cells (HCEC) encountered problems such as the fact that HCEC cells could only be seeded at high cell density (2000-5000 cells/square mm) therefore limiting the possibility to start a primary culture from small specimen, and that HCEC cells could not be passaged continuously at low seeding density (50-100 cells/square mm) which limits the ability to expand the HCEC stock for storage and future use.

### SUMMARY OF THE INVENTION

In a first aspect the invention relates to a method of non-enzymatic harvesting and *in vitro* culturing human corneal endothelial cells for transplantation comprising the steps of:
a) dissecting human corneal endothelial cells from a tissue source; growing said corneal endothelial cells at a low initial density of 100-500 cells/mm² in extracellular matrix coated (ECM) culture plates for a period of time, wherein said culture plates having been treated with a natural extracellular matrix as supplied by bovine corneal endothelial culture, or a synthetic attachment protein mixture, or growing said human corneal endothelial cells at a low initial density of 100-500 cells/mm² on a carbon plasma deposit known as diamond-like carbon (DLC);
b) passaging said cells into a secondary culture system wherein the secondary culture system is comprised of ECM coated culture plates and the addition of sufficient cellular growth factors;
c) growing the human corneal endothelial cells to confluency; and
d) harvesting the human corneal endothelial cells from the second culture system in sufficient quantities to be useful in transplantation to a subject in vivo.

The present invention provides for a method of culturing HCEC on a novel extracellular matrix which enables the establishment of primary cultures from small specimens of HCEC (100-500 cells). The present invention also provides for a method which to expand these primary HCEC colonies via serial passage into large quantity of cells for transplantation and cryostorage for future use.

A method for initiating primary cultures of corneal endothelial cells, including that of human origin, by using dissection enables the culture to start from a low initial density (100-500 cells/mm²) and expand from a seeding density of 1 to 32. These cells can be effectively passaged 7 to 8 times without losing their morphological integrity and physiological functions, such as formation of tight intracellular junctions and Na/K pump activation. The corneal endothelial cultures can be maintained in a commonly used fetal bovine serum (FBS) supplemented culture medium enriched with selected growth factors such as fibroblast growth factors 1 and 2 (FGF1, FGF2), epidermal growth factors (EGF), transforming growth factor β (TGFβ), endothelial cell growth factor (ECGF), and other growth factors known in the art of cell culture. In particular, if the corneal endothelial cells are propagated in a natural extracellular matrix as supplied by bovine corneal endothelial culture, or a synthetic attachment protein mixture containing such components as fibronectin, laminin, collagen type I and IV, and RGDS, or on a carbon plasma deposit known as diamond-like carbon (DLC), the culture will assume a more hexagonal morphology upon repeated passage at high split ratio (1:32 or 1:64) for up to 10 passages. The generation of a large pool of corneal endothelial cells, especially that of human origin, can be banked in cryo-storage and used for future cell transplantation procedures.

It is therefore an object of the present invention to provide a method of cell culture of HCEC that can be used for the establishing and serial culturing of other cell types of human origins such as neurons, pancreatic beta cells, and chrondocytes.

It is another object of the present invention to create HCEC in sufficient quantities of HCEC that can be used for other purposes.

It is a further object of the present invention to provide an in vitro cell culture model of the human cornea.

It is also an object of the present invention to provide a means for regenerating corneal endothelial cells in a cornea by replacing the damaged corneal endothelial cells with HCEC grown in culture system of the present invention.

It is yet a further aspect of the present invention to provide a means for regenerating other types of damaged human and mammalian endothelial cells by replacing the damaged endothelial cells with endothelial cells grown in the culture system of the present invention.

These and other objects of the invention, as well as many of the attendant advantages thereof, will become more readily apparent when reference is made to the following detailed description of the preferred embodiments.

### BRIEF DESCRIPTION OF THE FIGURES

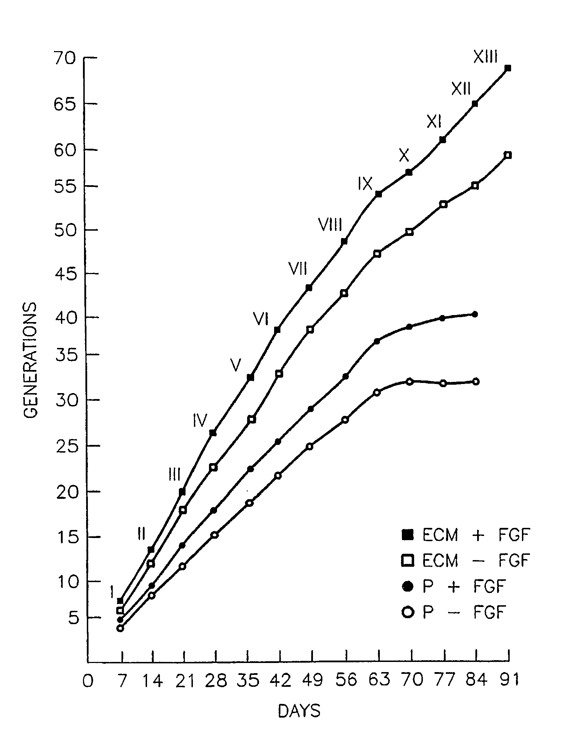

Figure 1 shows generation curves for long term serial propagation of cultured human endothelial cells on different substrates.

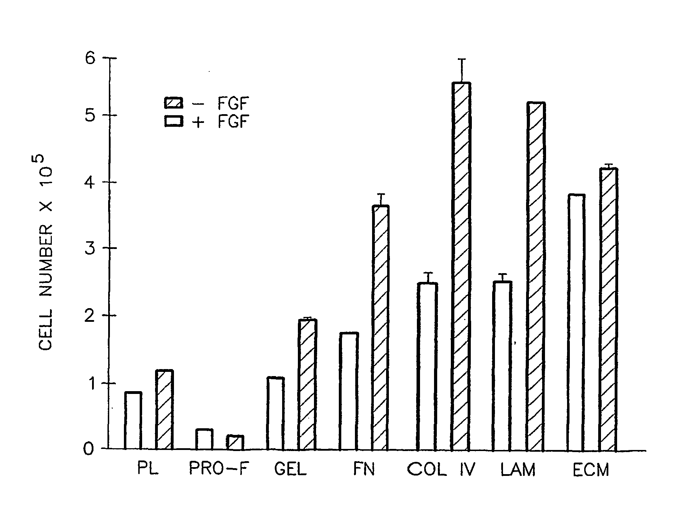

Figure 2 illustrates the effects of various attachment factors on the proliferation of cultured human corneal endothelial cells in the presence or absence of bFGF.

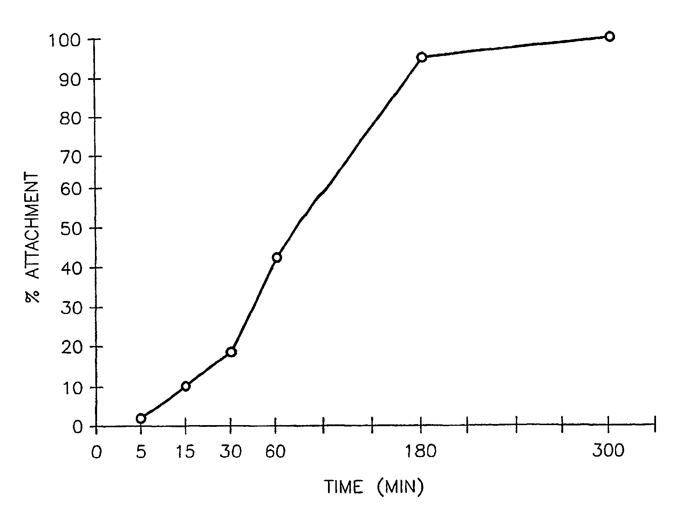

Figure 3 is a time curve of attachment of cultured human corneal endothelial cells onto the denuded human corneal buttons coated with attachment agents.

### DETAILED DESCRIPTION AND PREFERRED EMBODIMENTS

In the first step of constructing the in vitro cornea model, the endothelial cells are seeded onto membranes of a cell culture inset. These endothelial cells will form the inner layer, or basal layer, of the corneal equivalent.

We have developed a system where the native corneal endothelium from a donor cornea, whose endothelial cell population is inadequate in cell count or damaged due to disease or physical disruption, can be removed without harming the integrity of the Descemet's membrane and the structure and function of the stromal later. The preparation of such a denuded cornea can be achieved with treatment from a diluted detergent such as Triton X-100 (0.5-5%) or ammonium hydroxide (at concentrations ranging from 10 mM to 200 mM) for a time period ranging from 2 minutes to 60 minutes at a temperature of 4°C to 25°C. The denuded corneas will be washed extensively 8-10 times with phosphate buffered saline (PBS) to remove any detergent or ammonium hydroxide residue. The denuded corneas will then be ready for cell coating from the cultured human corneal endothelial cell stock.

Prior to seeding the cells onto the denuded corneal surface, a predetermined mixture of attachment proteins containing fibronectin (ranging from 0.1 µg to 500 µg/ml in PBS), laminin (0.1 µg to 500 µg/ml in PBS), RGDS (0.01 µg to 100 µg/ml in PBS), collagen type IV (ranging from 0.1 µg to 1000 µg in 0.1 M acetic acid) will be added to the denuded surface (Descemet's membrane) and incubated at 4°C for a period ranging from 5 to 60 minutes. The residual protein mixture will be removed after the incubation period, and the cornea is rinsed three times with PBS and placed endothelial side up on a Teflon concave holder. An 11 mm diameter button will be punched out with a size 11 trephine. This button will be ready to receive the cultured corneal endothelial cells.

The cultured human endothelial cells will be removed from the tissue culture dish with 0.05% trypsin and 0.02% EDTA in saline solution. The cell suspension will be counted with a Coulter Particle Counter (Z1 model, Beckman-Coulter) and a preparation of about 50,000 to 500,000 cells/ml, preferably about 200,000 cells in 200 µl of culture medium (DME-H16 with 5% fetal calf serum or a serum-free medium containing a mixture of attachment proteins such as fibronectin, laminin, and fibroblast growth factors (at 10 ng to 400 ng/ml) will be added carefully onto the denuded corneal button. A layer of 1% sodium hyaluronate, such as Healon® (Advanced Medical Optics, Santa Ana, CA) at approximately 0.1 to 0.5 ml, will be layered onto the cell suspension as a protectant. The corneal button will then be incubated at 37°C in a 10% CO₂ incubator for a period of 10 minutes up to 24 hours. Alternatively, the coated corneal button will be incubated for 20 minutes and the cornea will be rinsed three times with PBS at 25°C and ready for transplantation.

Alternatively, the process of maintaining human corneal endothelial cells in culture, expansion of the corneal endothelial cells, and the preparation of the attachment protein can be used to coat artificial cornea stroma generated from polymer-gel composition. Briefly, a poly-gel stroma can be molded into a cornea shape, and the concave side (endothelial side) will be treated with a mixture of attachment proteins and growth factors such as fibronectin (ranging from 0.1 to 500 µg/ml in PBS), laminin (ranging from 0.1 to 500 µg/ml in PBS), RGDS (ranging from 0.01 to 100 µg/ml in PBS), collagen type IV (ranging from 0.1 µg to 1000 µg in 0.1 M acetic acid), FGF (10 to 400 ng/ml in PBS), EGF (10 to 400 ng/ml in PBS), or TGFβ (1 to 100 ng/ml in PBS). After incubation at 4°C for a period ranging from 10 minutes to 2 hours, the artificial stroma will be rinsed three times with PBS, and cultured human corneal endothelial cells at a density of about 50,000 to about 10⁶ cells/ml preferably about 150,000 to 250,000 cells/200 ml of culture medium (DMA-H16 with 5% FCS or a mixture of attachment proteins containing fibronectin, laminin, RGDS, and collagen type IV) will be added to a corneal button of 11mm diameter. A layer of (10 mg/mL sodium hyaluronate, 0.1 to 0.5 ml) will be applied carefully onto the cell layer as a protectant, and the button will be incubated at 37°C in a 10% CO₂ incubation for a period ranging from 10 minutes to 24 hours. The artificial corneal button will be rinsed 3 times with PBS after the incubation and will be ready for transplantation.

In alternative embodiments, the corneal endothelial cells used to form the endothelial layer can be derived from a variety of mammalian sources. Non-transformed corneal endothelial cells derived from sheep, rabbit, and cows have been used. Mouse corneal endothelial cells have been transformed with large T antigen of SV40. (Muragaki, Y., et al., Eur. J. Biochem. 207(3):395-902 (1992).) Non-human cell types which can be used also include transformed mouse corneal endothelial cell lines, or normal corneal endothelial cells derived from sheep or rabbit. The normal rabbit endothelial cells can be derived from enzymatically dissociated corneal endothelium or from explants of cornea and are serially cultivated in MSBM medium (Johnson, W.E. et al., In Vitro Cell. Dev. Biol. 28A:429-435 (1992)) modified by the addition of 50 µg/mL heparin and 0.4 µg/mL heparin binding growth factor-1 (MSBME).

The use of endothelial cells from a non-corneal origin, is also disclosed. The non-corneal origin endothelial cells that have also been used in this invention include ovine and canine vascular and human umbilical vein endothelial cells. The endothelial cells may be transformed with a recombinant retrovirus containing the large T antigen of SV40 (Muragaki, et al., 1992, supra). Transformed cells continue to grow in the corneal equivalent and form mounds on top of the acellular layer due to their lack of contact inhibition. Non-transformed cells will form a monolayer underlying the stromal cell-collagen layer. Alternatively, normal endothelial cells may be transfected as above, but with the addition of a recombinant construct that expresses a heat sensitive gene. These transformed cells will grow in continuous culture under reduced temperature. After establishment of a confluent endothelial cell layer, the temperature can be raised to deactivate the transforming gene, allowing the cells to resume their normal regulation and exhibit contact inhibition, to form an endothelial cell monolayer similar to the non-transformed cells. Most peptides are heat sensitive (with the exception of heat shock proteins) so that there is a wide choice of peptides that can be deactivated by raising culturing temperature. Transformation in this way also facilitates the use of hard to obtain and cultivate cell types such as human corneal endothelial cells.

### Preparation of extracellular matrix (ECM) coated plates for primary corneal endothelial culture and subsequent passaging.

Bovine corneal endothelial cells (BCEC) in culture will be seeded onto dishes in a DME-H16 medium containing 10% FCS, 5% CS, 5% Dextran, 300 ug/ml glutamine, 2.5 ug/ml Amphotericin B, and 50 ng/ml bFGF. At confluency, (7-10 days post seeding), the dishes will be treated with 20 mM NH₄OH at a volume sufficient to cover at least 2/3 of the plate. After 5 minutes of shaking in a mechanical shaker, the NH₄OH will be aspirated and the dished rinsed 5 times with PBS. The dishes will be stored at 4°C at least a week prior to use in order to eliminate any surviving BCEC. Laminin and fibronectin will be dissolved in distilled water at a concentration of 100 µg/ml. Type IV collagen will be dissolved in 0.6% v/v acetic acid/water. Laminin, fibronectin, and type IV collagen will be added to the ECM plates as needed for culture purposes.

### Example 1: Non-enzymatic dissection of primary human corneal endothelial cells

The corneal rims from human donors (after the central portion has been removed for transplantation) or whole donor corneas will be rinsed in a large volume (50 ml) of phosphate buffered saline (PBS). They will then be placed in endothelial side up on a holder. The trabecular meshwork and remnants of iris will be removed carefully by micro-dissection. By using sharp pointed jeweler's forceps, the endothelial cell layers and the Descemet's membrane will be peeled off very carefully with great care taken not to include any underlying stromal tissue. This step can be confirmed by viewing the dissected Descemet's membrane under an inverted microscope to make sure it only carries the corneal endothelial cells on one side and nothing on the other side. The piece of tissue will be placed onto an ECM coated 35 mm tissue culture dish or similarly suitable container, filled with approximately 0.5 ml of culture medium (DME-H16 with 15% fetal calf serum enriched with b-FGF at 250 ng/ml). The dish will be incubated at 37°C in a 10% CO₂ incubator for 24 hours, and then another 1 ml of culture medium will be added. The sample will be incubated without disturbance for about 7 days to see if a colony of corneal endothelial cells migrates outwards from the tissue sample, at which time (7 to 14 days after the sample is placed in culture) the medium is changed every other day until the cell count reaches about 200-500 cells.

### Example 2: Culture of Human Corneal Endothelial Cells at High Split Ratio

When the primary cell count from the tissue sample outgrowth reaches a number of between 150 to 750, preferably between about 200 to 500, the cells will be released from the dish with STV solution (0.05% trypsin, 0.02% EDTA in normal saline). The STV solution will be removed when the cells round up but are still attached to the culture dish. No centrifugation step is necessary since the remaining STV will be inactivated by the growth media containing 15% fetal calf serum. The corneal cells will be placed onto a 60-mm ECM-coated dish (between about 250 to 1000 cells, preferably about 500 cells per dish). The medium will be changed every other day and b-FGF at a concentration of 250 ng/ml will be added at the time of medium change. At confluence (about 7 to 10 days after plating), the cells will be passaged again at the same split ratio (1:16 to 1:64) or will be frozen in 10% DMSO, 15% FCS at a density of between about 5 x 10⁵ to about 5 x 10⁶ cells/ml, preferably about 10⁶ cells/ml per ampoule and stored in liquid nitrogen for future use. The passaging can be carried out for up to 8 times without loss of cell functions or morphological integrity.

### Freezing of HCEC stock.

For each of the 5 ml of HCEC collected, 0.5 ml of DMSO was added to the cell suspension. Each 1.1 ml of the mixture was aliquoted into a 1.5 ml cryopreservation tube to yield an approximate 1 million cells per vial final concentration. The vials were then put into a Styrofoam box and let stand in a -80°C freezer for 24 hours. After 1 day, the ampoules were transfer into liquid nitrogen for long term storage.

### Example 3: Denudation of Corneal Button

Human donor corneal buttons are obtained from the Eye Bank. These corneal buttons are deemed unsuitable for transplantation due to inadequate endothelial cell counts, but otherwise are healthy and disease free and obtained under eye banking guidelines.

The corneal button will be placed endothelial side up in a holder, and rinsed three times with PBS. Then a solution of ammonium hydroxide at a concentration ranging from 10 mM to 200 mM will be added carefully into the corneal button without spilling over the top. The cornea will be kept at temperatures of about 10°C to 25°C for a period of 5 minutes up to 2 hours. Then the ammonium hydroxide will be removed, and the inside of the cornea button rinsed approximately 10 times with PBS. A cotton swab will be slid gently across the endothelial surface to remove any residual cell skeletons or debris. The corneal button is rinsed again three times with PBS, punched with an 11 mm trephine, and is then ready for coating with cultured human corneal endothelial cells.

Alternatively, the native corneal endothelium can be removed by adding Triton-X100 at a concentration of 0.5 to 5% in distilled water kept at 10°C for a period ranging from 5 minutes to 2 hours, and then processed as previously described. Furthermore, the corneal endothelium can be treated with distilled water for a period of 20 minutes to 2 hours at a temperature ranging from 4°C to 25°C. Then the cotton swab will be slid gently across the endothelial surface to remove the cell cytoskeleton and debris. The cornea will then be processed with an 11 mm trephination.

### Example 4: Treatment of Denuded Corneas with Attachment Proteins and Growth Factors.

After trephination, the denuded cornea button will be placed endothelial side up again in a holder. A solution of attachment proteins containing fibronectin at a concentration ranging from 10 µg to 500 µg/ml in PBS, laminin (10 µg to 500 µg/ml in PBS), RGDS (1 µg to 100 µg/ml in PBS), collagen type IV (10 µg to 1000 µg in 0.1 M acetic acid), b-FGF (1 to 500 ng/ml in PBS), EGF (1 ng to 500 ng/ml in PBS) will be added carefully onto the denuded cornea button. The specimen is allowed to incubate at 4°C for a time ranging from 5 minutes to 2 hours, at the end of which the cocktail will be removed and the cornea rinsed 3 times with PBS.

### Example 5: Coating of Denuded Cornea with Cultured Human Endothelial Cells

The cultured cornea endothelial cells will be removed from the culture dish with STV solution (0.05% trypsin, 0.02% EDTA in saline) as previously described. The cells will be centrifuged at 2000 rpm for 5 minutes and the culture medium removed. The cell pellet will be resuspended with 2 ml of DME-H16 medium containing fetal calf serum at a concentration of 0.1 to 5%. About 100 µl of the suspension will be counted in a Coulter Particle Counter to determine the cell number per ml. Then the cell concentration will be adjusted to about 5 x 10⁵ to 10⁷ cells/ml, preferably about 10⁶ cells/ml and 200 µg of the cell suspension will be added carefully added into the 11mm trephined corneal button. A layer of sodium hyaluronate, at a concentration of about 10mg/ml, ranging from 0.2 to 0.5 ml (Healon®) will be overlaid carefully onto the cell suspension as a protectant. The corneal button will then be incubated at 37°C in a 10% CO₂ incubator for a period of 20 minutes to 24 hours. The human corneal endothelial cell coated button will be ready for transplantation.

In an alternate embodiment, an artificial matrix can be generated although such material may not be as effective in promoting the growth and morphological integrity (hexagonal shape) of the HCEC.

To generate the artificial matrix, fibronectin, laminin and RGDS will be dissolved at concentrations of about 100 µg/ml in distilled water, and collagen type IV is dissolved at a concentration of about 1 mg/ml in 0.01% acetic acid. Basic FGF is dissolved at a concentration of about 100 µg/ml in bovine serum albumin (0.05% w/v). All the materials are mixed together in a 15 ml centrifuge tube and swirled gently to avoid bubbling. The mixture is then incubated at 4°C for two hours.

To coat the tissue culture dishes, the mixture is dilute 1:10 with phosphate buffered saline, and then 1 ml of the solution is added to a 35 mm dish and store at 4°C for 1 hour. Prior to use the solution is aspirated and the cell suspension will be added to the dish.

Having described the invention, many modifications thereto will become apparent to those skilled in the art to which it pertains without deviation from the invention as defined by the scope of the appended claims.

## Claims

1. An in vitro method of non-enzymatic harvesting and culturing human corneal endothelial cells for transplantation comprising the steps of:
a) dissecting human corneal endothelial cells from a tissue source; growing said corneal endothelial cells at a low initial density of 100-500 cells/mm² in extracellular matrix coated (ECM) culture plates for a period of time, wherein said culture plates having been treated with a natural extracellular matrix as supplied by bovine corneal endothelial culture, or a synthetic attachment protein mixture, or growing said human corneal endothelial cells at a low initial density of 100-500 cells/mm² on a carbon plasma deposit known as diamond-like carbon (DLC);
b) passaging said cells into a secondary culture system wherein the secondary culture system is comprised of ECM coated culture plates and the addition of sufficient cellular growth factors;
c) growing the human corneal endothelial cells to confluency; and
d) harvesting the human corneal endothelial cells from the second culture system in sufficient quantities to be useful in transplantation to a subject in vivo.

2. The method of claim 1 wherein the dissection of the corneal endothelial cells further comprises the step of removing the cells from the tissue source such that the cells are dissected away from the stroma of said tissue source prior to growing in the first culture system.

3. The method of claim 2 wherein said tissue source can be corneal buttons or rims.

4. The method of claim 2 wherein the ECM is comprised of Bovine corneal endothelial cell extracellular matrix (BCE-ECM) or an artificially generated extracellular matrix (AG-ECM).

5. The method of claim 4 wherein the artificially generated endothelial cell extracellular matrix (AG-ECM) is made by the process comprising:
a) fibronectin, laminin and RGDS (Arg-Gly-Asp-Ser peptide) are prepared in a 100 µg/mL in distilled water;
b) collagen type IV is prepared at a concentration of about 1 mg/mL in 0.01% acetic acid;
c) basic fibroblast growth factor (bFGF) is prepared at a concentration of about 100 µg/mL in bovine serum albumin (0.05% w/v);
d) the solutions of steps a, b, and c are mixed and then incubated at 4 °C for two hours; and
e) the mixture of step d is diluted about 1:10 with phosphate buffered saline, and then a sufficient amount of the solution is added to a dish and allowed to stand at 4 °C for approximately 1 hour before use.

6. The method of any of the preceding claims 1-5, wherein the corneal endothelial cells are grown at a density of 100-500 cells/mm².

7. The method of any of the preceding claims, wherein the cells are passaged at a high split ratio of 1:32 or 1:64.

## Patentansprüche

1. *In vitro* Verfahren eines nicht enzymatischen Erntens und Züchtens von Hornhautendothelzellen des Menschen für eine Transplantation, umfassend:
a) Präparieren von Hornhautendothelzellen des Menschen von einer Gewebequelle; Züchten der Hornhautendothelzellen mit einer anfänglich geringen Dichte von 100-500 Zellen/mm² in mit extrazellulärer Matrix (ECM) beschichteten Kulturplatten für eine Zeitdauer, wobei die Kulturplatten mit einer natürlichen extrazellulären Matrix behandelt wurden, wie sie durch eine Kultur von Hornhautendothelzellen des Rindes bereitgestellt wird, oder mit einem synthetischen Anhaftungsproteingemisch, oder Züchten der Hornhautendothelzellen des Menschen mit einer geringen anfänglichen Dichte von 100-500 Zellen/mm² auf einer Kohlenstoffplasmaabscheidung, die als diamantähnlicher Kohlenstoff (DLC) bekannt ist;
b) Transferieren der Zellen in ein zweites Kultursystem, wobei das zweite Kultursystem ECM beschichtete Kulturplatten und die Zugabe von ausreichenden Zellwachstumsfaktoren umfasst;
c) Züchten der Hornhautendothelzellen des Menschen bis zur Konfluenz; und
d) Ernten der Hornhautendothelzellen des Menschen von dem zweiten Kultursystem in ausreichenden Mengen, um bei einer Transplantation für ein Subjekt *in vivo* zweckdienlich zu sein.

2. Verfahren nach Anspruch 1, wobei das Präparieren der Hornhautendothelzellen weiterhin den Schritt eines Entfernens der Zellen aus der Gewebequelle umfasst, so dass vor dem Züchten in dem ersten Kultursystem die Zellen von dem Stroma der Gewebequelle wegpräpariert werden.

3. Verfahren nach Anspruch 2, wobei die Gewebequelle Hornhaut-Buttons oder -Randbereiche sein können.

4. Verfahren nach Anspruch 2, wobei die ECM eine extrazelluläre Matrix von Hornhautendothelzellen des Rindes (BCE-ECM) oder eine künstlich erzeugte extrazelluläre Matrix (AG-ECM) umfasst.

5. Verfahren nach Anspruch 4, wobei die künstlich erzeugte extrazelluläre Matrix der Endothelzellen (AG-ECM) durch ein Verfahren hergestellt wird umfassend:
a) Bereitstellen von Fibronektin, Laminin und RGDS (Arg-Gly-Asp-Ser Peptide) in 100 µg/ml destilliertem Wasser;
b) Bereitstellen von Collagen Typ IV in einer Konzentration von ungefähr 1 mg/ml in 0,01 %-iger Essigsäure;
c) Bereitstellen von basischem Fibroblasten-Wachstum-Faktor (bFGF) in einer Konzentration von ungefähr 100 µg/m in Rinderserumalbumin (0,05 % W/V);
d) Mischen und anschließend Inkubieren der Lösungen von Schritten a, b und c bei 4°C für zwei Stunden; und
e) Verdünnen des Gemisch von Schritt d ungefähr 1:10 mit Phosphat gepufferter Salzlösung, und anschließend Zugeben einer ausreichenden Menge der Lösung in eine Schale, wobei sie für ungefähr 1 Stunde vor der Verwendung bei 4°C stehengelassen wird.

6. Verfahren nach einem der vorstehenden Ansprüche 1-5, wobei die Hornhautendothelzellen mit einer Dichte von 100-500 Zellen/mm² gezüchtet werden.

7. Verfahren nach einem der vorstehenden Ansprüche, wobei die Zellen mit einem hohen Aufteilungsverhältnis von 1:32 oder 1:64 transferiert werden.

## Revendications

1. Méthode in vitro de prélèvement non enzymatique et de culture de cellules endothéliales cornéennes humaines pour une greffe comprenant les étapes de :
a) dissection des cellules endothéliales cornéennes humaines à partir d'une source de tissu : croissance desdites cellules endothéliales cornéennes à une faible densité initiale de 100 à 500 cellules/mm² dans des plaques de cultures enduites de matrice extracellulaire (MEC) pendant une certaine durée, lesdites plaques de culture ayant été traitées avec une matrice extracellulaire naturelle comme fourni par une culture de cellules endothéliales cornéennes bovines ou un mélange de protéines d'attachement synthétiques, ou croissance desdites cellules endothéliales cornéennes humaines à une faible densité initiale de 100 à 500 cellules/mm² sur un dépôt de plasma de carbone connu sous le nom de carbone sous forme de diamant amorphe (CDA) ;
b) passage desdites cellules dans un système de culture secondaire, le système de culture secondaire étant constitué de plaques de culture enduites de MEC et l'ajout de suffisamment de facteurs de croissance cellulaire ;
c) croissance des cellules endothéliales cornéennes humaines jusqu'à confluence ; et
d) prélèvement des cellules endothéliales cornéennes humaines du second système de culture en des quantités suffisantes pour une utilisation dans une greffe in vivo à un sujet.

2. Méthode selon la revendication 1, dans laquelle la dissection des cellules endothéliales cornéennes comprend en outre l'étape de retrait des cellules d'une source de tissu de sorte que les cellules soient disséquées loin du stroma de ladite source de tissu avant la croissance dans le premier système de culture.

3. Méthode selon la revendication 2, dans laquelle ladite source de tissu peut être des bords ou des boutons cornéens.

4. Méthode selon la revendication 2, dans laquelle la MEC est constituée d'une matrice extracellulaire de cellules endothéliales cornéennes bovines (MEC-ECB) ou d'une matrice extracellulaire générée de manière artificielle (MEC-GA).

5. Méthode selon la revendication 4, dans laquelle la matrice extracellulaire générée de manière artificielle (MEC-GA) est préparée par le procédé comprenant :
a) de la fibronectine, de la laminine et RGDS (peptide Arg-Gly-Asp-Ser) sont préparés à une concentration de 100 µg/ml dans de l'eau distillée ;
b) du collagène de type IV est préparé à une concentration d'environ 1 mg/ml dans de l'acide acétique à 0,01 % ;
c) un facteur de croissance des fibroblastes basique (FCFb) est préparé à une concentration d'environ 100 µg/ml dans de l'albumine sérique bovine (0,05 % m/v) ;
d) les solutions des étapes a, b et c sont mélangées, puis incubées à 4 °C pendant 2 heures ; et
e) le mélange de l'étape d est dilué à environ 1/10 avec une solution saline tamponnée avec du phosphate, puis une quantité suffisante de la solution est ajoutée à une boite et laissée au repos à 4 °C pendant environ 1 heure avant son utilisation.

6. Méthode selon l'une quelconque des revendications 1 à 5, dans laquelle les cellules endothéliales cornéennes sont cultivées à une densité comprise dans la plage allant de 100 à 500 cellules/mm².

7. Méthode selon l'une quelconque des revendications précédentes, dans laquelle les cellules effectuent des passages à une vitesse de division élevée de 1/32 ou 1/64.
